# EUROPEAN PATENT SPECIFICATION

(11) **EP 3 270 684 B1**
(45) Date of publication and mention of the grant of the patent: **05.08.2020**
(21) Application number: 16764352.7
(22) Date of filing: 18.03.2016
(51) Int. Cl.: A01G 31/00, A01G 9/20, A01G 31/04, A01G 9/24, A01H 1/02

(54) **A SYSTEM FOR INDOOR CULTIVATION OF PLANTS WITH SIMULATED NATURAL LIGHTING CONDITIONS**
SYSTEM ZUR INNENRAUMZÜCHTUNG VON PFLANZEN MIT SIMULIERTEN NATÜRLICHEN LICHTVERHÄLTNISSEN
SYSTÈME DE CULTURE EN INTÉRIEUR DE PLANTES DANS DES CONDITIONS D'ÉCLAIRAGE NATUREL SIMULÉ

(30) Priority: 19.03.2015 US 201562135514 P
(43) Date of publication of application: 24.01.2018
(73) Proprietor: Rokeha Ltd., 6901925 Kohav-Yaakov (IL)
(72) Inventor: KOP, Menachem, 4339814 Rananna (IL)
(74) Representative: Studio Torta S.p.A.
(86) International application number: PCT/IL2016/050300
(87) International publication number: WO 2016/147195

(56) References cited:
- WO-A1-2011/007868
- WO-A1-2012/157588
- WO-A1-2014/037852
- WO-A1-2015/123776
- CN-A- 103 929 949
- CN-A- 104 823 838
- CN-U- 201 766 924
- CN-U- 203 969 017
- DE-A1- 2 852 936
- GB-A- 2 431 328
- JP-A- 2011 200 148
- US-A- 4 594 811
- US-A1- 2008 110 088
- US-A1- 2011 061 297
- US-A1- 2014 000 162
- US-A1- 2014 000 162
- US-A1- 2014 208 647
- US-A1- 2015 181 821
- US-A1- 2015 296 727

## Description

### Field of the Invention

The present invention relates to the field of a plant cultivating system. More particularly, the invention relates to an indoor soilless plant cultivation system, for cultivating plants in a nutrient-rich solution.

### Background of the Invention

Many people are attracted to living in urban settings by virtue of the economic progress that may be realized. Cities bring together diverse groups of people and companies in ways that increase productivity and create the networks, clusters, and chance interactions that lead to the discovery of new innovations and the creations of new entrepreneurial businesses. Other advantages of living in urban settings include the large number of cultural activities that are available and the relative ease in commuting to work.

While 60% of the human population now lives in cities and are protected against the outdoor elements, food-bearing plants are subjected to the rigors of the outdoors. People hope for a good weather year in order to ensure that the food supply will be readily available. Many times due to a rapidly changing climate regime, however, massive floods, protracted droughts, class 4-5 hurricanes, and severe monsoons take their toll each year, destroying millions of tons of valuable crops.

By the year 2050, nearly 80% of the earth's population will reside in urban centers. Applying the most conservative estimates to current demographic trends, the Earth's population will increase by about 3 billion people during this period. An estimated 10.9 million square km of new land (about 20% more land than all of Brazil) will be needed to grow enough food to feed them, if traditional farming practices continue as today. At present, throughout the world, over 80% of the land that is suitable for raising crops is in use. Historically, some 15% of that agriculturally suitable land has been laid waste by poor management practices. Indeed, much land has become despoiled, such that natural eco-zones have been converted into semi-arid deserts.

The traditional agricultural practice of growing food-bearing plants outdoors, or within greenhouses located at agricultural areas, is problematic in terms of weather related or pest related crop failure, the cost of transporting the grown crops to food distribution centers, the ecological damage due to fossil fuel emissions from the vehicles that transport the crops and that are used for performing agricultural activities such as plowing, the cost for fertilizers and pesticides, the occurrence of infectious diseases acquired at an agricultural interface, and ecological damage due to agricultural runoff.

In order to sustain the Earth's growing population, it would be desirable to learn how to safely grow food within city-located, environmentally controlled multistory facilities, in order to maintain a readily available food supply while overcoming the problems associated with traditional agricultural practices.

Some indoor hydroponic system are known from the prior art wherein plant growth units are stacked one on top of another, a solution of water and plant nutrient is introduced to the plants, and panels comprising artificial light sources that eliminate the need for natural sunlight and enable light cycles of varied duration are provided on top of each plant growth unit.

Photoperiodic flowering plants flower in response to a sensed change in night length, and therefore require a continuous period of darkness before floral development can begin. However, the prior art light panels for simulating such light cycles are costly due to the need of a light panel at each level of a growth unit and of a dedicated control system for each panel. Additionally, the light panels are self-heating, and expensive to operate cooling systems are needed to remove the generated heat.

Light-emitting diodes (LEDs) have been found to be ideal light sources for crop production by virtue of their small size, durability, long operating lifetime, wavelength specificity, relatively cool emitting surfaces and linear photon output with electrical input current. Work at NASA's Kennedy Space Center has focused on the proportion of blue light required for normal plant growth as well as the optimum wavelength of red and the red/far-red ratio. The addition of green wavelengths for improved plant growth has also been addressed. ["Plant Productivity in Response to LED Lighting", G. Massa et al, HortScience, December 2008, vol. 43, no. 7, 1951-1956]

However, the inability of such prior art lighting systems to provide substantially equal light distribution limits implementation thereof for an indoor plant growth unit of large vertical dimensions.

Another drawback of prior art systems for cultivating plants is the safety of workers, when pest control is needed, in which case the entire cultivating space is sprayed by pesticide. This implies using a greater amount of pesticide. However, some pesticides may cause cancer and other health problems, as well as harming the environment.

An indoor soilless plant cultivating system is known from US 2014/0000162 A1.

It is an object of the present invention to provide an indoor soilless cultivating system for the sustainable crop production of a safe and varied food supply.

It is an additional object of the present invention to provide an indoor soilless cultivating system with a lighting system that maintains a substantially equal light distribution to facilitate photosynthesis at an indoor plant growth unit of relatively large vertical dimensions.

It is an additional object of the present invention to provide an indoor soilless cultivating system by which the operating and capital costs of light sources used to simulate the light cycles required by photoperiodic flowering plants are significantly reduced relative to those of the prior art.

It is yet an additional object of the present invention to provide an indoor soilless cultivating system by which the operating and capital costs of cooling systems for removing the heat generated by light sources that simulate the cyclical nature of natural sunlight are significantly reduced relative to those of the prior art.

It is yet another object of the present invention to provide an indoor soilless cultivating system which saves a substantial amount of the required pesticide to be sprayed, to thereby reduce the exposure of workers and the environment to harmful effects.

Other objects and advantages of the invention will become apparent as the description proceeds.

### Summary of the Invention

The present invention provides an indoor soilless plant cultivating system according to claim 1.

According to the invention the system comprises a drive unit for cyclically rotating each of the towers so as to be sequentially exposed to morning light conditions, noon light conditions, afternoon light conditions and nighttime conditions in accordance with the illumination signature emitted by the light posts of the at least one module. The drive unit is configured to cause a complete tower rotation once every 24-hour period.

Each of the towers is preferably configured with a plurality of mounting elements by each of which a corresponding plant is mountable at a different tower peripheral portion and is urged to grow outwardly from said peripheral portion, groups of said mounting elements being defined at different height levels of the tower.

Leaves of all of the plants being grown on one of the towers are exposed to a substantially uniform distribution of light emitted from light elements mounted on an adjacent one of the light posts for a given emulated time period despite a height differential between the plants.

To achieve the substantially uniform distribution of light, the light elements may be sufficiently small such that they have a density of no less than 40 light elements within a light post height of 50 cm and are mounted on each of the light posts in such a way that only one light element is mounted at any given height. A segment of the light elements has a predetermined number and sequence of light elements arranged such that constituent beams emitted from the light elements of said segment are mixed within a conical distribution angle to provide a photosynthetic photon flux density at the tower peripheral portion upon which the mixed beam impinges that stimulates photosynthesis for a given plant being grown. Thus the photosynthetic photon flux density at another tower peripheral portion being illuminated at the given emulated time period is substantially equal.

The predetermined number and sequence of light elements are preferably repeated along the height of the light post for all other segments.

According to the invention each of the light elements is provided with a directional lens configured to produce a light emitting angle whose angular boundaries are incident on the tower periphery, causing propagation of the emitted light to an internal region of the module between two adjacent towers to be blocked as a result of its incidence on the tower periphery, to thereby ensure that said internal region will be darkened to a radiation level less than a predetermined photosynthetically active radiation level for the plant being cultivated.

The plant cultivating system provides at least the following advantages:
- A modular scalable system that is simple to ship, build and maintain.
- The system can be deployed in any existing building with any geometrical shape regardless of its original purpose.
- Dynamic allocation of the number of towers inside the same facility, or on different floors of the same facility, for different crops depending on seasonal demand or opportunities.
- The facility is isolated from outdoor conditions to support plant cultivation every hour and every day of the year regardless of the outdoor weather conditions and climate.
- Substantial shortening of the growth cycle of each plant, for extremely fast growth of high quality products.
- The number of plants able to be grown in the system for a given area is 7 times greater as compared to traditional hydroponic growth.
- Operation of the system approaches an optimum point in combining usage of light, air, water which are the most critical elements conducive to plant growth.
- The plants being grown are not subject to damage due to extreme meteorological conditions and natural disasters.
- Crops have maximum nutritional values, superior taste and freshness.
- Reduced refrigerated transportation time and cost.
- As the cultivating system is soilless, 95% less water is required to grow the crops than prior art systems.
- No greenhouse gas emissions.
- Easy and relatively inexpensive closed perimeter security and surveillance systems, preventing agricultural theft losses that are on the rise worldwide.
- No soil pollutants.
- Solution of land shortage problem.
- Airflow system by which plant-released carbon dioxide is transported to a daytime region for an improved photosynthesis process.
- Artificial pollination.

### Brief Description of the Drawings

In the drawings:
- Fig. 1 is a plan view of a plant cultivating system, according to one embodiment of the present invention;
- Fig. 2 is a plan view of a plant cultivating system, according to another embodiment of the invention;
- Figs. 3A and 3B are a schematic side view of two light posts, respectively, showing the relative arrangement of the light elements mounted thereon;
- Fig. 3C is a schematic illustration of the conical distribution angle of light that is emitted from a light element segment of a light post and that impinges upon a peripheral tower portion;
- Fig. 4 is a schematic illustration in elevation view of one embodiment of irrigation means for irrigating plants being hydroponically cultivated;
- Fig. 5 is a schematic illustration in elevation view of one embodiment of irrigation means for irrigating plants being aeroponically cultivated;
- Figs. 6A and 6B are schematic illustrations in elevation view of another embodiment of irrigation means for irrigating plants being aeroponically cultivated;
- Fig. 7 is a front view from within the interior of a portion of an outer wall of a tower used in conjunction with the irrigation means of Fig. 1;
- Fig. 8 is a schematic illustration of a recycling system for efficiently utilizing the irrigation fluid used in conjunction with the plant cultivating system;
- Fig. 9 is a schematic illustration in side view of a temperature of a closed-loop liquid circulation system air to control the temperature of air in the vicinity of a tower;
- Fig. 10 is a schematic illustration of an air circulation arrangement used in conjunction with the plant cultivating system for facilitating an increase in plant growth;
- Fig. 11 is a schematic illustration of an artificial pollination system used in conjunction with the plant cultivating system;
- Fig. 12 is a perspective view from the side of structural elements for use in conjunction with a module of towers;
- Fig. 13 is a perspective view from the top of the structural elements of Fig. 12, showing an upper frame and a centrally positioned ceiling fan;
- Fig. 14 is an enlarged perspective view from the side of the upper frame of Fig. 13, showing one embodiment of a drive unit for rotating a tower;
- Fig. 15 is an enlarged perspective view from the side of a tower wall of Fig. 13, showing a removable plant supporter;
- Fig. 16 is a perspective view of a multidirectional spraying column;
- Fig. 17 is a plan view of a module of towers, showing the relative position of the multidirectional spraying column of Fig. 16; and
- Fig. 18 is a schematic illustration of a control system used in conjunction with the plant cultivating system for modulating the light energy directed to the plants.

### Detailed Description of Preferred Embodiments

The present invention is an energy efficient, indoor soilless plant cultivating system which employs a plurality of stationary light posts, each of which illuminates a predetermined sector of an indoor facility in accordance with a predetermined illumination signature. The plants to be cultivated are mounted on a plant growth unit provided with irrigation means (hereinafter "tower") of a large vertical dimension similar to that of each light post, for efficiently utilizing the inner dimensions of the facility, which may be an abandoned building in an urban setting or a building in an industrial park dedicated to be used by the cultivating system. The system operates in conjunction with a module that includes a predetermined number of towers, such that each tower of a module is rotated by a drive unit about a vertical axis in accordance with a predetermined timing sequence so as to be exposable to the light generated at any given time by one or more of the light posts. An interior region of the module is not exposed to the light generated by any of the module related light posts, and the plants instantaneously positioned within the darkened interior region receive the sensation of nighttime.

The indoor facility is preferably isolated from the outdoor conditions, including light, humidity and temperature conditions, present outwardly from the facility. The plant cultivating system is able to emulate optimal outdoor growing conditions that are different from the instantaneous outdoor conditions, so that the leaves of all plants subjected to a controlled environment will be exposed to a substantially uniform light distribution for the given emulated time period despite a height differential between therebetween. Even though the plants are isolated from the outdoors, the production of fruit and seed crops is made possible by virtue of an artificial pollination system.

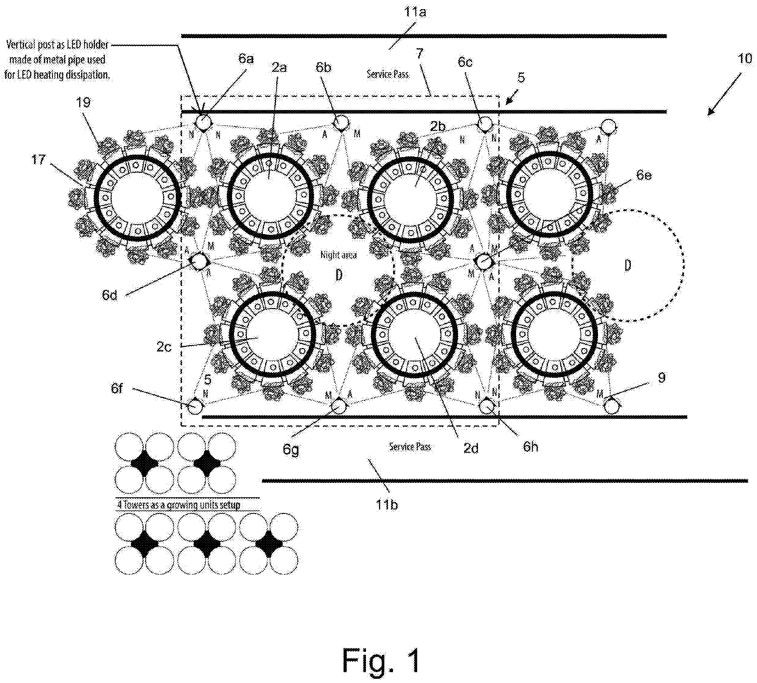

Fig. 1 schematically illustrates a plant cultivating system 10 in plan view, according to one embodiment of the present invention. Plant cultivating system 10 comprises a plurality of modules, and for purposes of brevity, one of the modules 5 will be described.

Module 5 includes four circular towers 2a-d arranged in a symmetrical square-like configuration. Eight evenly spaced light posts 6a-h are deployed adjacent to the imaginary perimeter 7 of module 5, such that first row light posts 6a-c are positioned adjacent to adjoining service pass 11a, third row light posts 6f-h are positioned adjacent to adjoining service pass 11b which is opposite to service pass 11a, and second row light posts 6d-e are positioned at the two sides, respectively, of perimeter 7 according to the illustrated orientation, while being positioned at an intermediate region of module 5 and interposed between a first row and third row light post.

Service passes 11a and 11b, to be used for accessing the towers for maintenance, plant treatment and harvesting purposes, may have a width of 70 cm. Harvesting may be carried out with manual carts that are advanceable along rails. The carts may have a hydrologic raising capability to permit comfortable access to an upper tower region. For use during extreme cold weather conditions, a rail may be configured as a series of interconnected round hollow pipes through which warm water is flowable, to support heat dispersion as a part of the ambient control system of the facility. These pipes may have a unique mechanical profile, for example funnel-shaped, to assist in uniformly spreading the heat.

Each of the light posts may operate continually, to emit light in accordance with a predetermined post-specific illumination signature, along a predetermined angular sector S, e.g. 60 degrees. The setting of the predetermined angular sector may be obtained by means of a directional lens 9 provided with each light element mounted on a post and by a selected spacing between a light element and a corresponding lens. Each light element also has a designed illumination range.

In the exemplary deployment of the light posts, the illumination signature of posts 6a, 6c, 6f and 6h simulates the lighting conditions of noontime at a region N. The instantaneous illumination signature of posts 6b, 6d, 6e and 6g simulates the lighting conditions of morning at a region M, and afternoon or evening at a region A, with respect to light intensity and/or wavelength. Darkened interior region D is located beyond the limited illumination range of the lighting elements mounted on each of light posts 6a-h, and therefore plants instantaneously positioned within darkened region D receive the sensation of nighttime. A distance between the towers at a darkened region D may be 80 cm for towers having a diameter of 60 cm.

Each of towers 2a-d has mounting means 17 by which each corresponding plant 19 is retained on the periphery of a tower while being exposed to the light posts. The various plants are arranged in layers, so that plants 19 are found throughout the height and circumference of a tower, for maximum utilization of the volume within the facility. Plants 19 may also be arranged in an inclined disposition, so that will be urged to grow outwardly from the tower without interfering with an adjacent plant.

The plant cultivating system of the present invention is conducive to the growth of many different types of crops, particularly high quality crops that are not necessarily indigenous to the surroundings of the given facility by virtue of the optimal environment in which they are grown, including leafy vegetables such as lettuce, chicory, tomato, cucumber, chili, pepper and spinach, berries such as strawberries, cranberries, blueberries and raspberries, and herbs such as herbs for flavoring, food, medicine and cosmetics, for example medical cannabis.

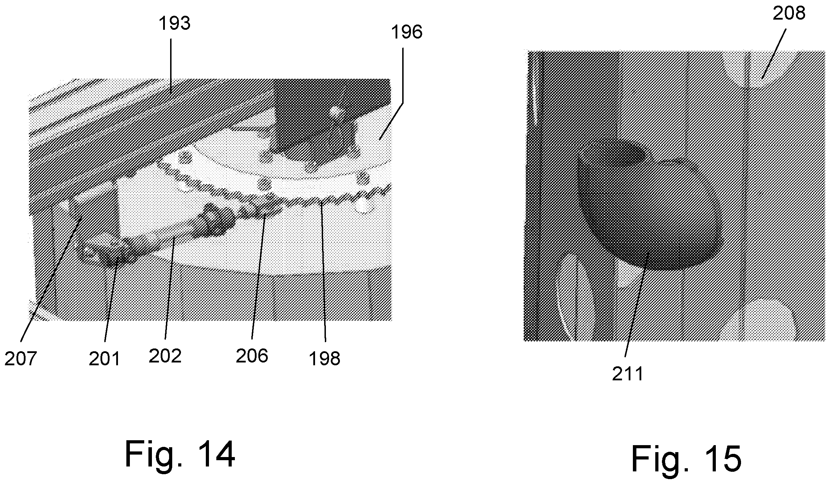

The circular configuration of the towers promotes trellising of climbing plants such as cherry tomatoes and grave vines around the tower periphery to advantageously minimize usage of the module surface area. Removable supporters 211 (Fig. 15) may be plugged into vacant holes 208 around the tower periphery to support the weight of the crop if the load on the tower is anticipated to be excessive.

Directional lens 9 is configured to produce a light emitting angle whose angular boundaries, when taking into account the given tower diameter and the given distance from a light post to a tower, are tangential with, or are otherwise incident on, the periphery of the tower. The propagation of the emitted light to an internal region of module 5 is blocked as a result of its incidence on the tower periphery, to thereby ensure that the internal region between two adjacent towers will be darkened to a radiation level less than a predetermined photosynthetically active radiation level for the plant being cultivated, for example darkness levels of up to 90% or more. The darkness level is also assisted by the ongoing growth of the leaves or branches of the plants which help to block the penetration of light into the inner region.

The rotation of each of towers 2a-d by means of a central vertical shaft and a drive unit allows each plant 19 to be cyclically exposed to morning light conditions, noon light conditions, afternoon light conditions and nighttime conditions by completing a full rotation about its vertical axis once every 24-hour period, thus simulating a daily day/night cycle. The drive unit may be an electric motor, or a hydraulically or pneumatically actuated drive unit.

It will be appreciated that a tower need not rotate at a constant rate. If a selected plant flourishes when exposed to certain light conditions, the relative dwelling time of the plant in those optimum lighting conditions may be increased.

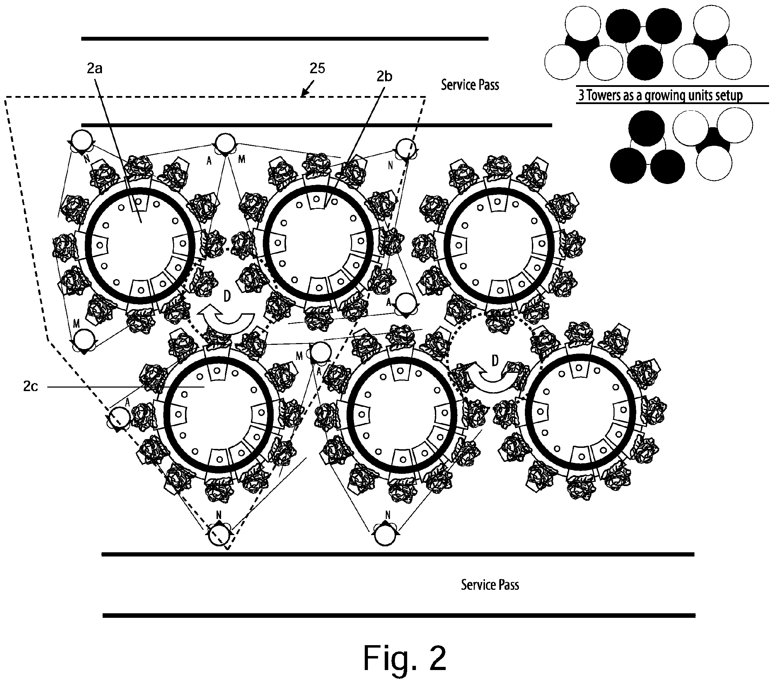

Fig. 2 illustrates a module 25 comprising three rotatable towers 2a-c, providing darkened interior region D, to which the mounted plants are cyclically exposed, as described above.

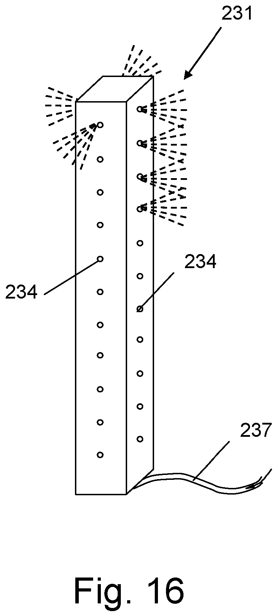

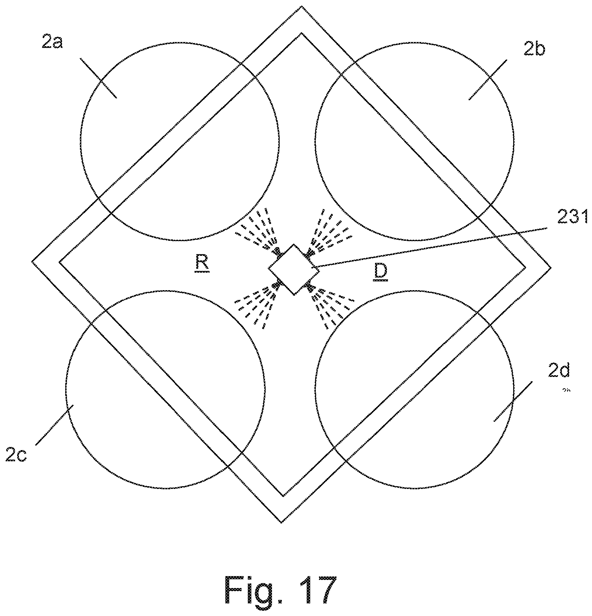

The definition of the darkened interior regions by the aforementioned module configurations advantageously contributes to the safety of workers and other bystanders by deploying a multidirectional spraying column 231 illustrated in Figs. 16 and 17 within a darkened region D.

Multidirectional spraying column 231, which may have a rectilinear or curvilinear configuration, has a plurality of nozzles 234 that protrude in different directions. When pesticide is delivered through conduit 237, for example in response to a controlled duty cycle via an underground conduit, to spraying column 231, a spray is issued from each nozzle 234 and is directed at each of towers 2a-d. The extending direction and the spray pattern of each nozzle 234 are carefully selected to avoid pesticide wastage as a result of unnecessary spraying in a region R between towers.

Since towers 2a-d are continuously rotated, all plants will be exposed to the sprayed pesticide. However, workers are generally located within service passes 11a-b (Fig. 1) which are outwardly separated from the modules, and will therefore not be exposed to the sprayed pesticide. This spraying arrangement will increase the safety of workers and will significantly reduce, or substantially eliminate harm, to the environment by minimizing discharge of harmful pesticide. Also, the amount of pesticide needed for effective pest control will be significantly reduced.

Even though the plants are grown in a soilless environment and are therefore not susceptible to damage by soil dwelling pests, nevertheless Small plantings that were germinated outside the facility and were already infected by pests or bacteria before being mounted in a tower, and therefore need to be treated with pesticide.

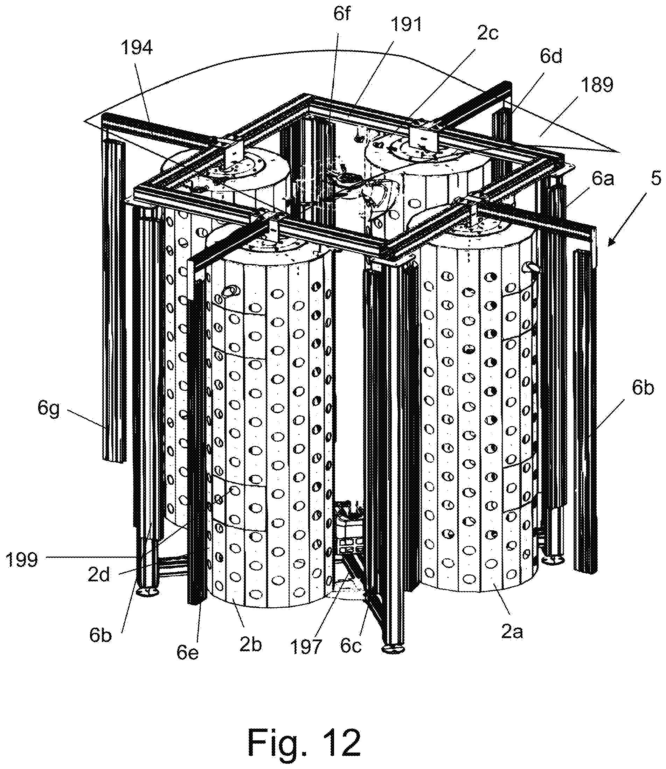

Figs. 12-15 illustrate exemplary structural features for use with the plant cultivating system.

As shown in Fig. 12, the vertical shaft of each of the four towers 2a-d of module 5 is rotatably mounted from above in a corresponding seat or bearing provided in an upper square or rectangular frame 191 and from below in a corresponding seat or bearing provided in a bottom bar 197. Upper frame 191 may be embedded in a roof or ceiling portion 189, or may be internally open and positioned below roof or ceiling portion 189.

The eight stationary light posts 6a-h are attached to upper frame 191 and are in fixed contact with the underlying ground surface, light posts 6a, 6c, 6f and 6b extending downwardly from a corresponding corner of the upper frame and the remaining light posts being connected to a corresponding cross member 194 extending outwardly from a central region of an upper frame side element. Each of the light posts is thus positioned at a relatively short and defined distance from the periphery of a tower, for example a minimal distance between the light post and tower periphery of 30 cm, although this distance is generally reduced due to the presence of the growing leaves. While the tower rotates, the actual distance from a plant to the light post varies. The four bottom bars 197 extend inwardly from a bottom portion of each of the light posts underlying a corresponding corner of upper frame 191 and are connected together.

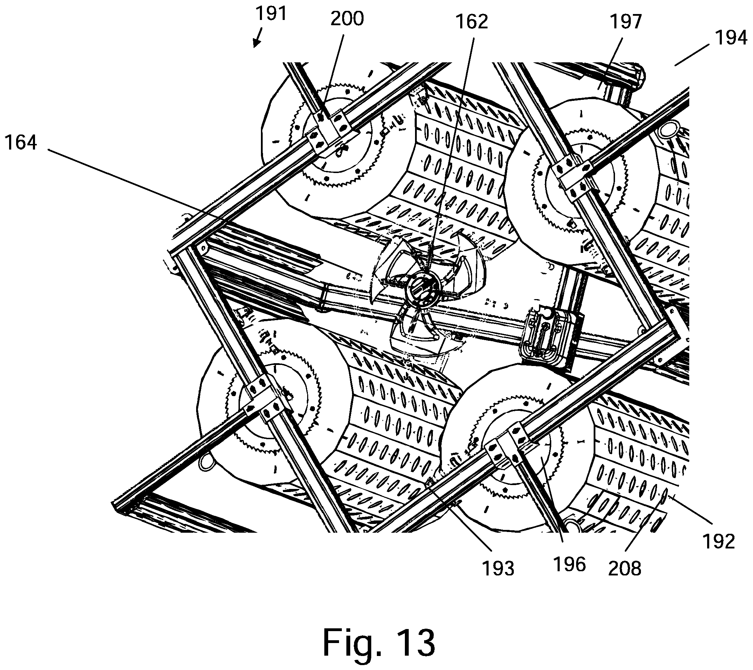

The central opening of upper frame 191 facilitates the positioning of ceiling fan 162 (Fig. 13), the purpose of which will be described hereinafter. Ceiling fan 162 may be suspended by a hangar attached to an overlying ceiling region, so as to be positioned within the central opening, whether at, above or below the height of upper frame 191. Alternatively, the grille 164 of fan 162 may be connected to two or more side elements 193 of upper frame 191.

Each tower 2a-d may be configured with one or more access hatches 199 that cover a corresponding opening formed in the periphery of a tower. The hatches 199 enable maintenance workers to access the hollow core of a tower, in order to clean or repair the tower periphery and the irrigation elements, for example, or for harvesting purposes. The hollow core also facilitates the growth of plants with large sized tubers and bulbs.

As shown in Fig. 13, each tower may be configured with a polygonal periphery that is substantially circular, such that each vertically extending and planar wall 192 defines a wall of the polygon. A plurality of vertically spaced planting holes 208 by which a plant is mounted on the tower are formed within each wall 192. If a plant has a size which is not compatible with the opening of a planting hole 208, a supporter 211 shown in Fig. 15, e.g. configured as an elbow made of molded rubber or plastic, may be removably inserted in one of the planting holes to assist in securely mounting a differently sized plant.

For example, a tower configured with a height of 240 cm and a diameter of 57 cm was formed with 11 planting holes 208 in each wall 192, when each planting hole was spaced by 20 cm center to center from an adjacent planting hole on the same wall.

Walls 192 may be made of an opaque or black material for optimal light absorption. The inner surface of a wall 192 may be provided with grooved drainage channels, e.g. vertically extending, by which irrigation fluid is directed towards the roots of the plants, to thereby maximize usage thereof.

One embodiment of the drive unit is shown in Figs. 13 and 14. A serrated wheel 196 is fixed to the upper surface 197 of each of the towers, so as to be coaxial therewith. The longitudinal axis of serrated wheel 196 is rotatably mounted at the corresponding junction 200 between upper frame side element 193 and cross member 194, to facilitate rotation of the tower.

A terminal end 201 of a substantially horizontally disposed reciprocating piston rod assembly 202, which may be hydraulically, pneumatically or electrically actuated, is connected, e.g. pivotally connected, to bracket 207 extending downwardly from side element 193. Piston rod assembly 202 has a bifurcated head 206 that is adapted to receive within its interior a tooth 198 radially extending from the periphery of serrated wheel 196, when the piston rod is extended, and to apply a force to a side edge of tooth 198, causing serrated wheel 196 and the tower connected thereto to rotate about its vertically oriented longitudinal axis for a discrete angle depending on the predetermined stroke of the piston rod. The piston rod is then retracted, in anticipation of an additional rotation initiating operation.

In order to ensure substantially homogeneous distribution of the light emitted by the elongated light posts onto vertically spaced plants, which may be spaced along a common tower by a large difference in height of as much as 3 meters or more, the light elements are densely mounted on each light post, for example 50 light elements are mounted within a distance of 50 cm such that only one light element is mounted at any given height. A number and sequence of light elements may be pinpointed in order to generate a plant-specific light signature that will optimize plant growth.

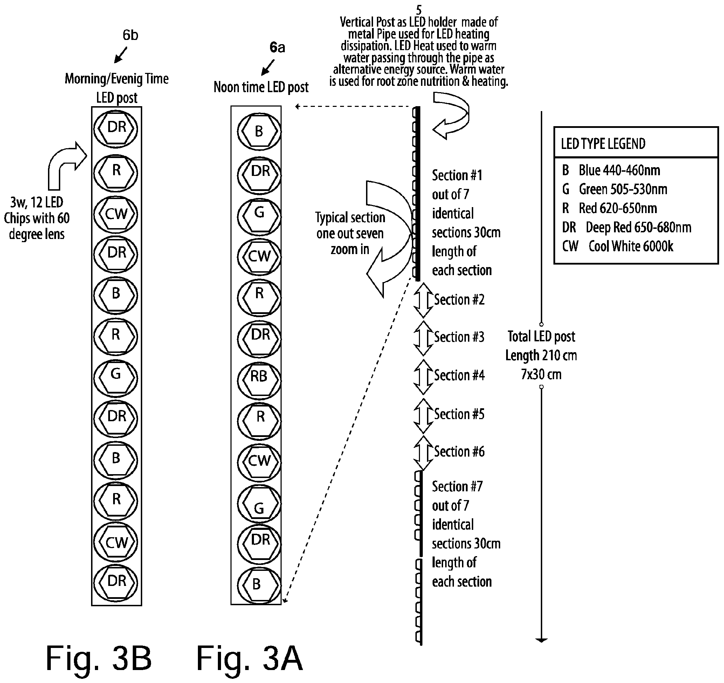

Figs. 3A and 3B schematically illustrates an exemplary sequence of the light elements, which are shown in exaggerated size for clarity and are mounted on light posts 6a and 6b for generating an illumination signature that emulates the lighting conditions of noontime and of reduced light intensity conditions, respectively. The light elements, which are vertically spaced and vertically aligned, are preferably LED elements, although other light elements are also in the scope of the invention. Each light post is preferably tubular, to maximize heat dissipation from the continually operating light elements. If so desired, the light elements may be operated according to a selected duty cycle or time sequence, in order to generate a desired waveform.

Light elements for emitting the following five colors are illustrated: blue (B) at a wavelength of 440-460 nm for use mainly during noon conditions, , green (G) at a wavelength of 505-530 nm, red (R) at a wavelength of 620-650 nm for use mainly during morning/afternoon conditions, deep red (DR) at a wavelength of 650-680 nm, and cool white (CW) at a color temperature, or the temperature of an ideal black-body radiator that radiates light of a comparable hue, of 5000°K. These colors were selected as they constitute the basic spectral components of sunlight needed by plants, although other colors are also in the scope of the invention.

The sequence of the light elements is carefully selected so as to generate a desired plant-specific light signature as a result of the interaction of the light beams emitted from adjacent light elements and of the vertical wavelength distribution throughout the length of the light post. The light signature generated by two adjacent light posts is also able to interact.

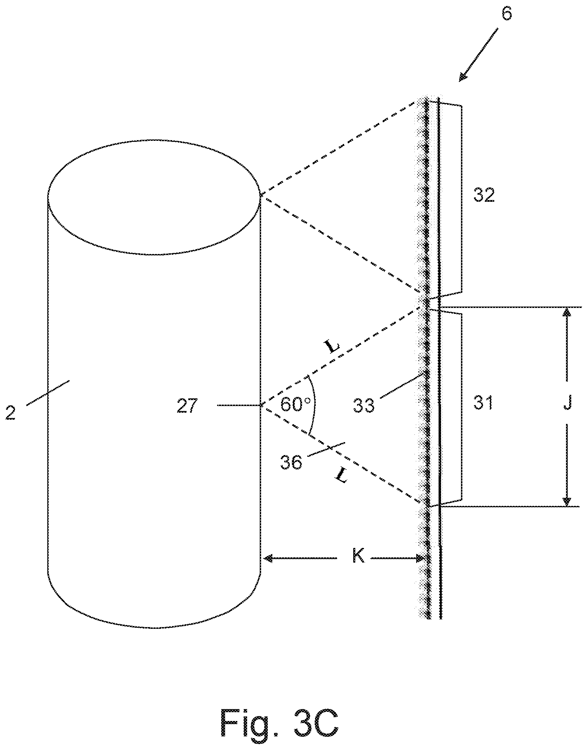

A segment 31 of light elements 33 having a height J is shown in Fig. 3C. Segment 31 includes a predetermined number of vertically spaced light elements 33, for example 30 elements. Each light element 33 of segment 31 emits a corresponding beam that impinges upon a peripheral portion 27 of tower 2, which is spaced by a distance K from light post 6. Within the conical distribution angle 36 of light that is emitted from segment 31, being bounded by equal sides L to define an isosceles triangle in cross section, the constituent beams emitted from each light element 33 are mixed to provide a photosynthetic photon flux density (PPFD) at peripheral portion 27 that optimally stimulates photosynthesis for the given plant being grown. Likewise the PPFD at any other peripheral portion 27, or at any leaf of the plant being cultivated which is adapted to absorb the emitted light, included within conical angle 36 is substantially equal. The light element sequence of segment 31 is repeated along the height of light post 6 for all other segments, for example segment 32 adjacent to segment 31. This light element arrangement thus promotes substantially equal light distribution to all plants being grown throughout the height of tower 2.

Morning and afternoon lighting conditions may be emulated, for example, by generating the following percentage of relative light energy corresponding to spectral components of light emitted from a light element segment: (1) dark blue at a wavelength of approximately 450 nm, 12%, (2) red at a wavelength of approximately 660 nm, 62%, (3) infrared at a wavelength of approximately 730 nm, 7%, and (4) white at a color temperature of 4000°K, 19%. Plants are generally exposed to these morning and afternoon lighting conditions for two quarters of a 24-hour period.

Noon lighting conditions may be emulated, for example, by generating the following percentage of relative light energy corresponding to spectral components of light emitted from a light element segment: (1) dark blue at a wavelength of approximately 450 nm, 34%, (2) red at a wavelength of approximately 660 nm, 31%, (3) infrared at a wavelength of approximately 730 nm, 7%, and (4) white at a color temperature of 4000°K, 28%. Plants are generally exposed to these noon lighting conditions for one quarter of a 24-hour period.

The tubular configuration of the light posts may also be utilized to enable circulation through their interior of an irrigation fluid. The irrigation fluid flowing through the sealed interior of a light post cools the continually operating light elements, and in turn becomes heated to plant growth inductive temperature of approximately 30°C. The heated irrigation fluid in turn is directed to the plants, for fostering their growth. The normally unexploited energy source of heat dissipated from light sources is therefore utilized to improve the plants' growth.

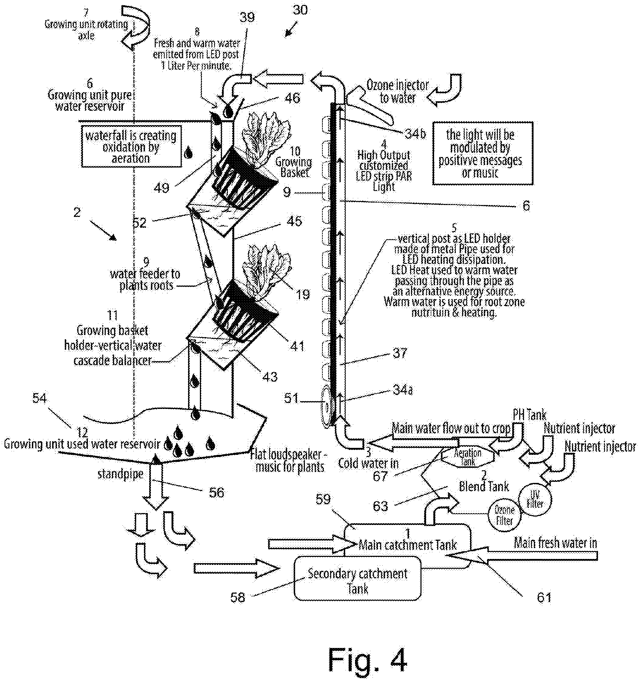

Fig. 4 illustrates one embodiment of irrigation means 30 for watering the plants which are being hydroponically cultivated. Cold irrigation fluid 34a is injected into the interior 37 of light post 6 and is progressively heated as it rises within the light post interior. The warm irrigation fluid 34b is discharged from the top of light post interior via emitter 39 to reservoir 46 located at the top of tower 2, for example at a rate of 1 L/min.

Each plant 19 being cultivated is retained in a basket 41 that allows the roots to be exposed to the irrigation fluid. Basket 41 is turn is mounted in a corresponding inclined hollow holder 43, e.g. cylindrical, which is secured to, or integrally formed with, the vertical outer wall 45 of tower 2, allowing each plant 19 to suitably grow while being exposed to the light emitted from elements 9.

A corresponding conduit 49 extends downwardly, for example at an incline, from reservoir 46 to a holder 43, or from a first holder to a second holder therebelow, to introduce the irrigation fluid to each holder. As each holder 43 is disposed at an incline with respect to vertical wall 45, the accumulation 52 of the introduced irrigation fluid is collected at the bottom of the holder at a height suitable for the immersion therein of the roots of plant 19 so as to supply nutrients to the plant, and then overflows in cascaded fashion to the holder therebelow. The spent overflow eventually flows to reservoir 54 at the bottom of tower 2. Each conduit 49 may be semicircular so as to simulate oxidation within the cascading irrigation fluid while being exposed to the surrounding air.

The effluent from bottom reservoir 54 flows through standpipe 56 to a secondary catchment tank 58 and then to main catchment tank 59 by gravity, to which fresh water is added via inlet 61. A blend tank 63 receives the discharge from main catchment tank 59. Additives, such as nitrogen, phosphorus, potassium, and other essential nutrients normally found in soil, are added, in optimum concentrations and in correct balance, are added. An aeration pump 67 delivers the produced irrigation fluid 34 to the inlet of the light post interior 37.

A sound emitter 51, e.g. a loudspeaker, may be mounted on the outer wall of light post 6, for generating acoustical signals that may be conducive for the plant growth.

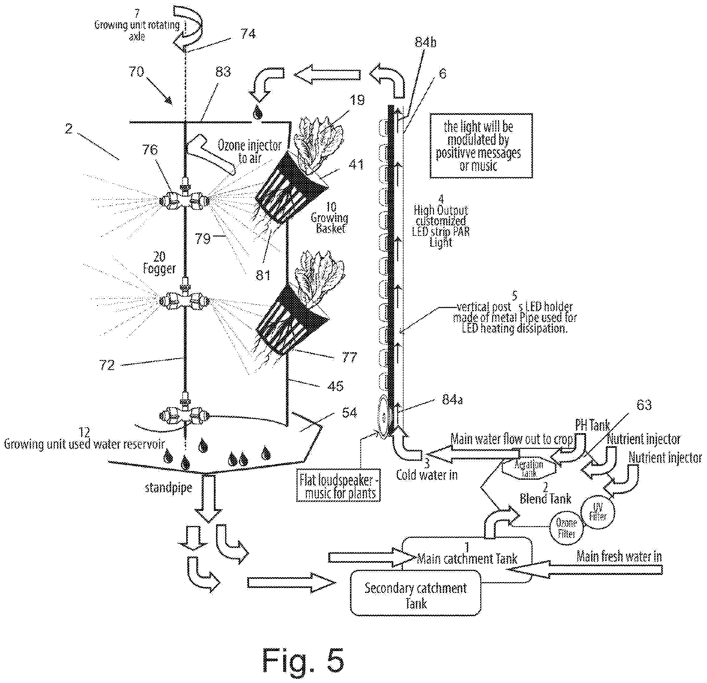

Irrigation means 70 illustrated in Fig. 5 may be used for watering plants 19 which are being aeroponically cultivated. Irrigation fluid is introduced from blend tank 63 to pipe 72 formed within the interior of vertical shaft 74 by which tower 2 rotates. The blending of the irrigation fluid is similar to that described in Fig. 4. A plurality of vertically spaced foggers 76 mounted on vertical shaft 74 and in fluid communication with pipe 72 eject a mist 79 directed to the roots 81 of plants 19 for providing a plentifully supply of oxygen. Each plant 19 being cultivated is retained in a basket 41 which is fitted within an aperture formed in vertical wall 45 of tower 2, and is mounted at an incline by means of a corresponding oblique brace 77, thereby allowing roots 81 to be exposed to the irrigation fluid.

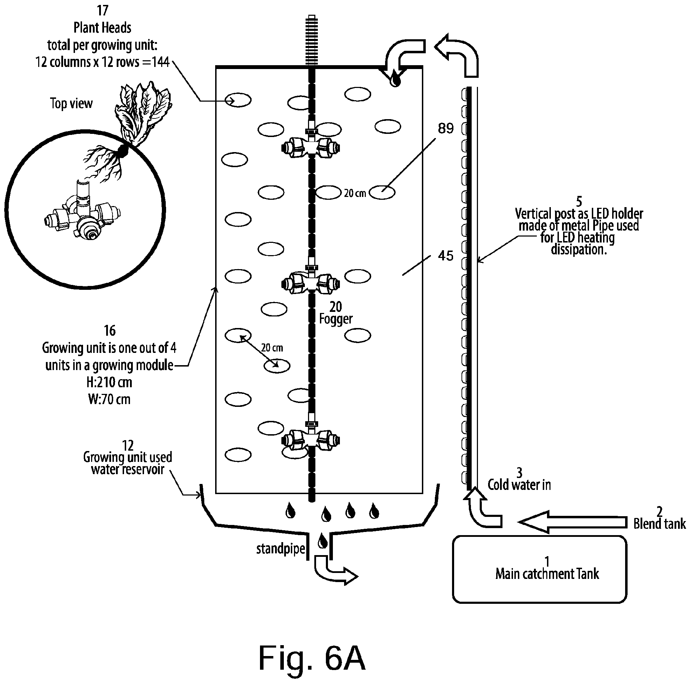

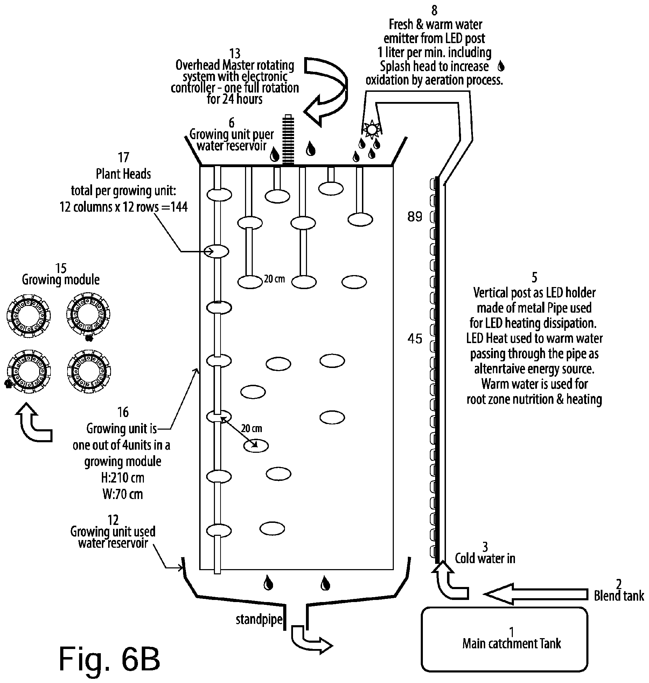

An exemplary arrangement of apertures 89 formed in outer tower wall 45 is shown in Figs. 6A and 6B.

At the same time, rotation of shaft 74 according to a predetermined timing sequence causes plants 19 to be exposed to the light generated at any given time by one or more of light posts 6. The interior of each light post interior is cooled by injected cold water 84a that is progressively heated as it rises. The heated water 84b is discharged through top plate 83 of tower 2 and is collected at bottom reservoir 54.

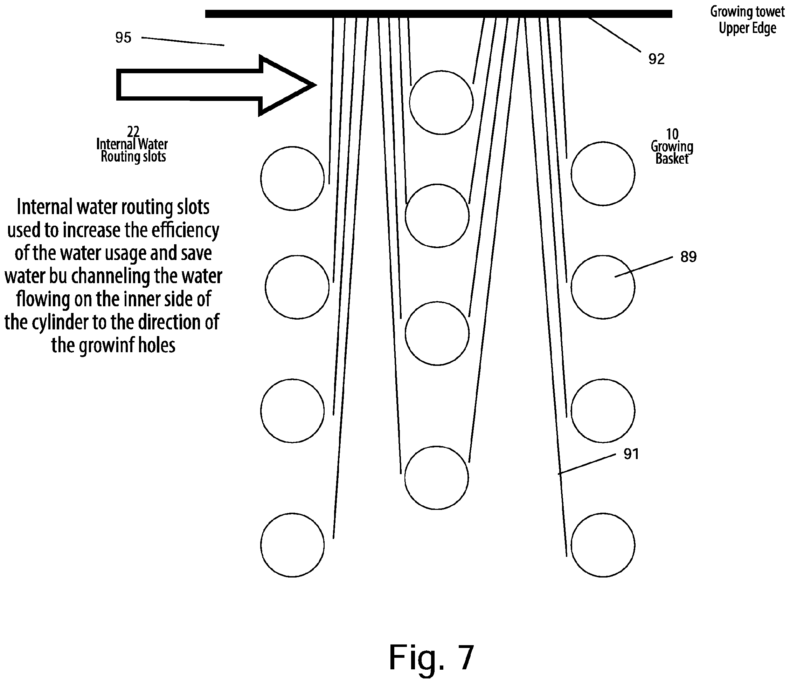

Fig. 7 illustrates a configuration of an outer wall 95 of the plant growth tower by which water conservation is considerably increased. Outer wall 95 is formed with a plurality of narrow slots 91, each of which is preferably recessed in order to receive liquid discharge from a fogger that has impacted the outer wall and would normally flow downwardly into the bottom reservoir of the tower without irrigating a plant. Each slot 91 extends for example from upper edge 92 of outer wall 95 to the periphery of an aperture 89 into which a plant growing basket is fitted, thereby providing another source of irrigation in addition to the fogger discharge. A slot 91 need not be straight as shown, but rather may be curved, or assume any other desired shape or disposition.

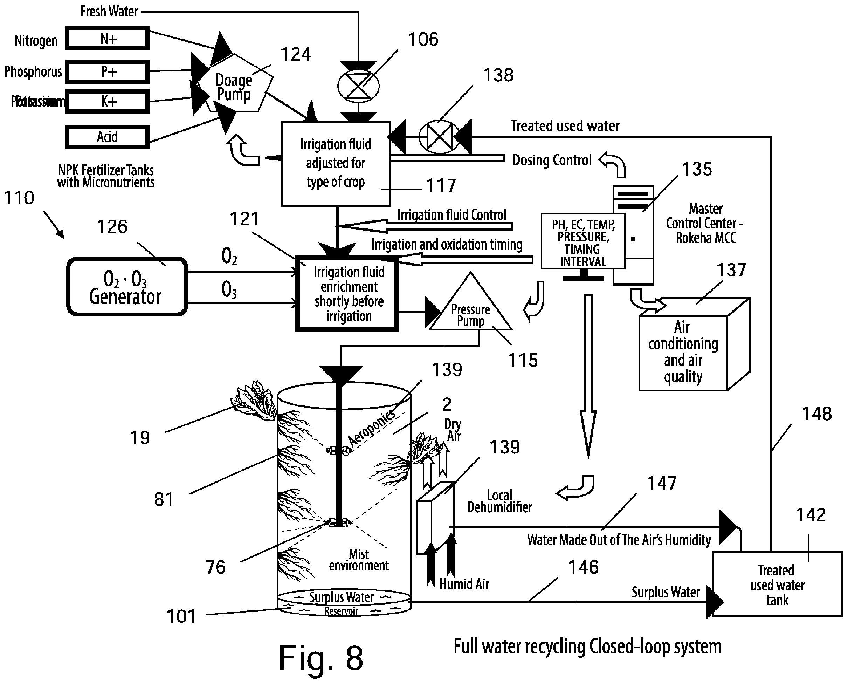

Fig. 8 schematically illustrates an open-loop recycling system 110 for efficiently utilizing the irrigation fluid. In system 110, the roots 81 of plants 19 retained within the interior of tower are aeroponically irrigated by means of the irrigation fluid that is delivered by high pressure feed pump 115 through central vertical pipe 72 of tower 2 and to vertically spaced foggers 76, to produce a mist environment.

The irrigation fluid is fed to feed pump 115 from second mixing chamber 121, into which is introduced the discharge of both first mixing chamber 117 and ozone generator 126, the latter serving to inject an oxidizing agent in the form of O2 or O3 into irrigation fluid for the purpose of disinfecting waterborne organisms and thereby enriching the fluid. In first mixing chamber 117 are mixed fresh water flowing through valve 106, e.g. a control valve, and the discharge of dosage pump 124, e.g. a peristaltic dosing pump, which delivers a predetermined amount of nutrients, such as nitrogen, phosphorus, potassium, and acid, needed by the type of crop being cultivated.

Controller 135 is in data communication with feed pump 115, dosage pump 124, and ozone generator 126, in order to regulate the conductivity and pH of the irrigation solution and to deliver it to foggers 76 at predetermined times. Ozone generator 126 is generally commanded to operate shortly before the activation of feed pump 115, to ensure suitable oxygenation of the irrigation fluid. Controller 135 may also be in data communication with air conditioning system 137 and local dehumidifier 139 for maintaining a predetermined air quality, including a desired degree of humidity, in the vicinity of each tower 2.

The surplus irrigation fluid not consumed by the plant roots 81 is collected in a reservoir 101 at the bottom of tower 2. A condensate pump, upon being commanded by controller 135 at a predetermined time, delivers the collected irrigation fluid via conduit 146 to used fluid storage tank 142, which also receives condensate delivered from dehumidifier 139 via conduit 147. A recirculation pump in data communication with controller 135 delivers the reused fluid to first mixing chamber 117 via conduit 148 and valve 138, which may be a control valve commanded by controller 135.

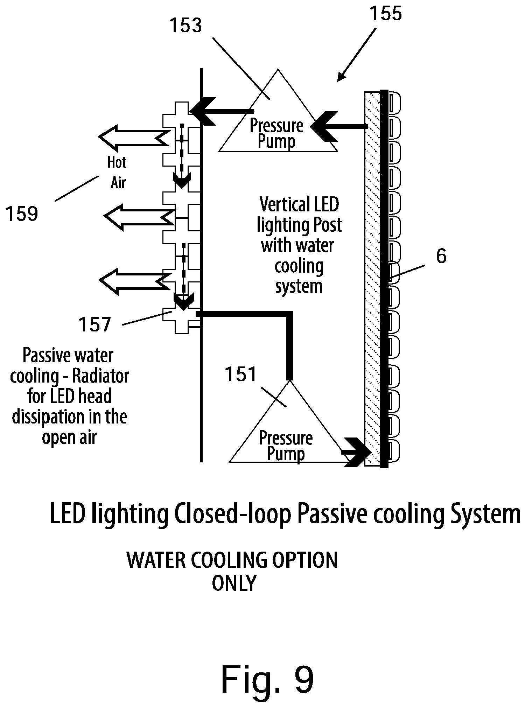

In addition to air conditioning system 137 and dehumidifier 139 (Fig. 8), the temperature of air in the vicinity of each tower may be controlled by means of the closed-loop liquid circulation system 155 shown in Fig. 9. Pump 151 delivers the cooling liquid upwardly through the interior of light post 6 to become progressively heated while the continually operating light elements mounted on the light post become cooled. The heated irrigation fluid discharged from the top of light post interior is pressurized by pump 153, and consequently flows at a sufficiently high rate through liquid-air heat exchanger, e.g. a radiator, to cause the surrounding air 159 to become heated. The heat depleted liquid is then introduced to pump 151. The increase in temperature of the surrounding air 159 may be controlled by the flowrate of the circulating liquid.

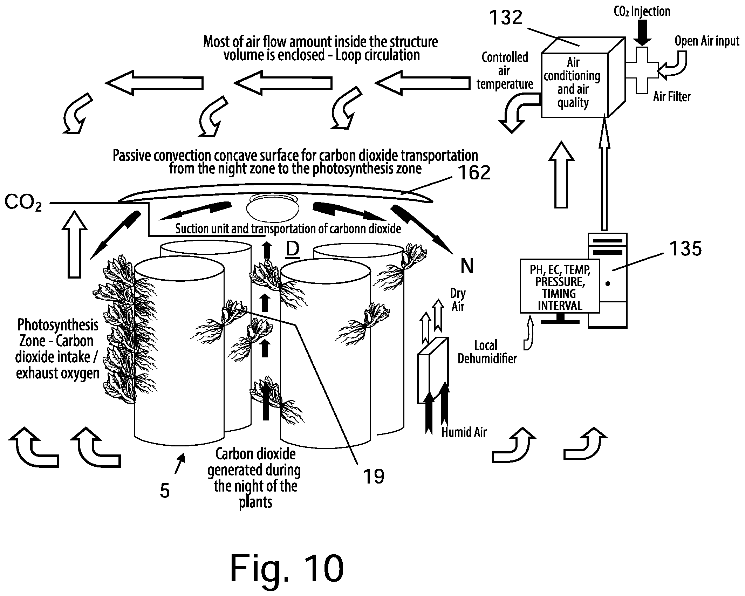

Fig. 10 illustrates an air circulation arrangement that facilitates an increase in plant growth. A ceiling fan 162 is installed so as to be centrally positioned within, and above, darkened interior region D of module 5. Since plants 19 release carbon dioxide during respiration at night, darkened interior region D is characterized by an increased concentration of carbon dioxide relative to other regions of the module. During operation of ceiling fan 162, the plant-released carbon dioxide, or air saturated with the plant-released carbon dioxide, is subjected to suction by ceiling fan 162, and is accordingly caused to be transported to outer noontime regions N of module 5, or alternatively to morning or afternoon regions. Plants 19 require a significant amount of carbon dioxide in order to conduct photosynthesis. By being able to direct the normally unexploited source of plant-released carbon dioxide to a daytime region, plants 19 are advantageously able to undergo an increased rate of growth while producing a larger amount of sugars and carbohydrates during the photosynthesis process as a result of absorbing a corresponding increased amount of carbon dioxide. Ceiling fan 162 may be deactivated when it overlies a region that is instantaneously illuminated with daytime light conditions.

The photosynthesis process is accompanied by loss of water as a result of evaporation from the stomates, or microscopic openings in the leaves of a plant through which incoming and outgoing gases such as carbon dioxide and oxygen and water vapor are released. The transport of plant-released carbon dioxide to a daytime region, resulting in a larger degree of photosynthesis, thus contributes to an even greater rate of water evaporation, inducing the plant in response to absorb a correspondingly increased amount of water through its roots to maintain an optimal water balance. The plant may also be induced to absorb an increased amount of water through its roots by commanding dehumidifier 139 (Fig. 8) to maintain a relatively low moisture level in the plant growing space surrounding a tower relative to the high moisture level within the core of a tower.

The intake of water through the roots of a plant is a major driving force for the movement of minerals from the roots and the transport of photosynthesis derived sugars throughout the plant. The plants grow in an optimal soilless environment at a controlled temperature and humidity, and consume a very small amount of water relative to their outdoor cultivated counterparts. As the roots do not have to expend the plant's energy to penetrate soil in quest for water and nutrients, the unused energy can be utilized by the plant's metabolic processes in other ways. For example, fruits tend to be sweeter, while leafy vegetables achieve a crispy leaf texture since the plant utilizes the unused energy to produce more minerals.

It will be appreciated that the plant-released carbon dioxide may also be transported through ducts, for example connected to upper frame 191 (Fig. 13), to a daytime region.

The temperature of the transported carbon dioxide, as well as fresh air, if desired to be mixed therewith, may be controlled by air conditioning system 137 as commanded by controller 135.

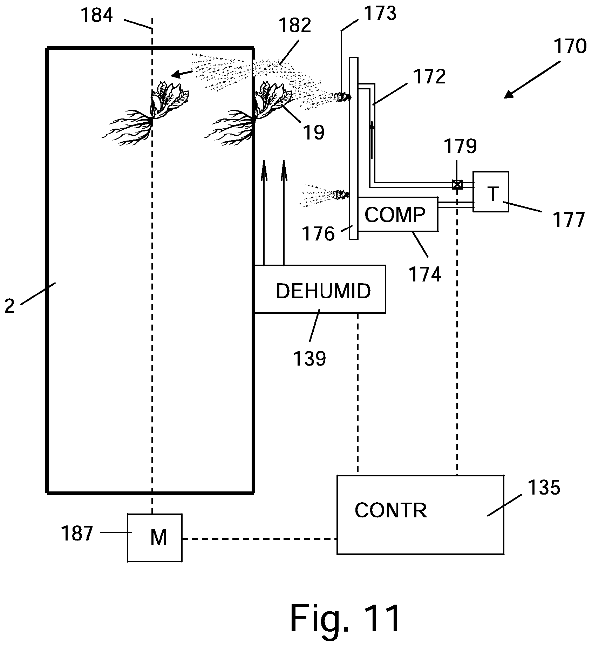

In another embodiment, an apparatus which is not of the present invention may be used in conjunction with artificial pollination system 170 shown in Fig. 11. Light post 176 carries a plurality of vertically spaced air discharge nozzles 173 which receive a pulsed supply of compressed air in parallel from air receiver tank 177. Air receiver tank 177 for storage of compressed air in turn is in fluid communication with compressor 174, positioned at a region of low humidity and possibly positioned on the floor of the facility. Compressor 174 is activated when the pressure within tank 177 is less than a predetermined low value, and is deactivated when the pressure within tank 177 is greater than a predetermined high value. A conduit 172 external to light post 176 extends from tank 177 and is in fluid communication with each nozzle 173, and a control valve 179 may be operatively connected with conduit 172, adjacent to the outlet port of tank 177. Each nozzle 173 may have a diverging outlet to direct the discharged compressed air in a conical pattern, to ensure impingement of the compressed air onto the stamen of plant 19, for example a strawberry plant, to induce the release of pollen 182 from its anther and the airborne transport of pollen 182 to the carpel of the same or of an adjacent plant.

Artificial pollination system 170 of course is capable of inducing the release of pollen from its anther only when the pollen bearing plant is reliably positioned in close proximity to a nozzle 173 at substantially the same height. Repeated and reliable rotational displacement of tower 2 about its longitudinal axis 184 may be made possible by a step motor 187, which is adapted to rotate tower 2 in discrete predetermined step increments in response to a command pulse received by the driver circuit. Alignment of a plant with a corresponding nozzle 173 may be achieved by knowing the angular displacement of each step, the diameter of the tower and the number of plants that are mounted around the circumference of the tower.

The efficacy of artificial pollination system 170 may be enhanced by a controlled change in the local humidity. Controller 135 is therefore operable to perform the five stage process of (1) commanding dehumidifier 139 to significantly reduce the local humidity in the vicinity of tower 2, for example to a level of 20% for strawberries, to reduce the adhesiveness of the pollen and to thereby support release of the pollen bearing anther from the stamen filament, (2) receiving information from the driver circuit of motor 187 as to when, or as to how many steps are made, until a given plant 19 will be positioned in pollen releasable proximity to nozzle 173, (3) commanding opening of control valve 179 for a predetermined time so that the compressed air will be directed to a given plant, (4) commanding dehumidifier 139 to significantly increase the local humidity in the vicinity of tower 2 following the anther release, for example to a level of 50% for strawberries, to ensure viability of the pollen and the adhesiveness of the stigma on which the pollen is to be deposited, and (5) closing control valve 179 at the conclusion of the pollination cycle.

The duration of the control valve opening may be regulated by controller 135 in response to the instantaneous air pressure within air receiver tank 177, to ensure a sufficiently high air flowrate to induce the release of pollen from its anther. For example, each nozzle 173 may be spaced 30 cm from the tower periphery, and the pressure of air when being discharged from the nozzle is about 6 bar, regardless of the number of nozzles.

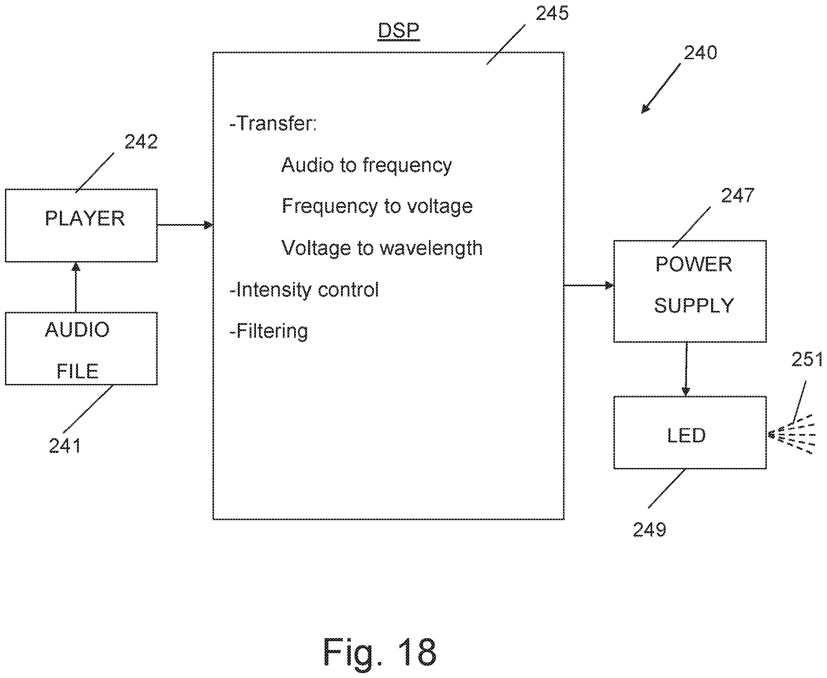

Fig. 18 illustrates another embodiment of the invention wherein the light emitted to the plants is modulated.

Several studies conducted by Dr. T.C. Singh, head of the Botany Department at Anamalia University, India and others confirmed that the music affects plant growth. Plants feel the vibration of the generated sound waves, and will speed the protoplasmic movement in the cells, to stimulate the manufacture of more nutrients that will give a stronger and better plant. [http://hubpages.com/living/the-effect-of-music-on-plant-growth, updated on November 12, 2015, 10/03/2016]

Control system 240 directs modulated light energy to the plants to stimulate an improvement in metabolic processes similarly to a plant reaction to modulated acoustic waves. The modulated light energy is generated by a digital signal processing (DSP) module 245 configured with a suitable transfer function, which may be housed in controller 135 (Fig. 10) or in any other suitable hardware component. In response to the input of an audio file 241 transmitted by player 242, DSP module 245 transfers the audio signal to discrete frequency components, and then these frequency components are sequentially transferred to modulated voltage components and modulated light wavelength components to generate a corresponding light waveform. DSP module 245 also controls the light intensity of the light waveform, depending on the daylight region to which the plants are presently exposed, and filters the light waveform. The output light waveform is transmitted to the programmable power supply 247 of the LEDs 249 mounted on a light post to generate the desired modulated light beam 251.

In another embodiment, all planting holes formed in the tower walls are assigned a unique identifier which is stored in a system database. The following information related to each plant being grown is associated with the identifier and is also stored in the database: time of planting, growing protocol parameters, geographical location at any given time, and time of harvesting. The precise real-time geolocation of every plant with respect to a service passage facilitates the use of robotics for plant harvesting.

While some embodiments of the invention have been described by way of illustration, it will be apparent that the invention can be carried out with many modifications, variations and adaptations, and with the use of numerous equivalents or alternative solutions that are within the scope of persons skilled in the art, without exceeding the scope of the claims.

## Claims

1. An indoor soilless plant cultivating system (10) for emulating outdoor growing conditions, comprising:
a) a plurality of stationary light posts (6a-h), each of which adapted to illuminate a predetermined sector of an indoor facility in accordance with a predetermined illumination signature;
b) a plurality of plant growth towers (2a-c) that are exposable to the light generated at any given time by one or more of the light posts (6a-h) and that are arranged by at least one module defining a module darkened interior region (D) within which instantaneously positioned plants (19) receive a sensation of nighttime;
c) a plurality of light elements mounted on each of said light posts (6a-h) wherein each of said light elements is provided with a directional lens (9) configured to produce a light emitting angle whose angular boundaries are incident on a tower (2a-d) periphery, causing propagation of corresponding emitted light to said interior region (D) of the module (5) between two adjacent towers (2a-d) to be blocked as a result of its incidence on the tower (2a-d) periphery, to thereby ensure that said interior region (D) will be darkened to a radiation level less than a predetermined photosynthetically active radiation level for the plant (19) being cultivated;
d) a drive unit configured to cyclically rotate each of said towers (2a-d) about a substantially vertical axis by a complete tower rotation once every 24-hour period so as to be sequentially exposed to morning light conditions, noon light conditions, afternoon light conditions and nighttime conditions in accordance with the illumination signature emitted by the light posts (6a-h) of the at least one module (5) ; and
e) irrigation means for supplying the plants (19) being cultivated in each of said towers (2a-d) with a nutrient-rich solution.

2. The system (10) according to claim 1, wherein each of the towers (2a-d) is configured with a plurality of mounting elements by each of which a corresponding plant (19) is mountable at a different tower peripheral portion (27) and is urged to grow outwardly from said peripheral portion, groups of said mounting elements being defined at different height levels of the tower (2a-d) .

3. The system (10) according to claim 2, wherein leaves of all of the plants (19) being grown on one of the towers (2a-d) are exposed to a substantially uniform distribution of light emitted from the light elements (33) mounted on an adjacent one of the light posts (6a-h) for a given emulated time period despite a height differential between the plants. plants (19).

4. The system (10) according to claim 3, wherein the light elements (33) are sufficiently small such that they have a density of no less than 40 light elements (33) within a light post height of 50 cm and are mounted on each of the light posts (6a-h) Z in such a way that only one light element (33) is mounted at any given height.

5. The system (10) according to claim 4, wherein a segment (31) of the light elements (33) has a predetermined number and sequence of light elements (33) arranged such that constituent beams emitted from the light elements (33) of said segment (31) are mixed within a conical distribution angle to provide a photosynthetic photon flux density at the tower peripheral portion (27) upon which the mixed beam impinges that stimulates photosynthesis for a given plant (19) being grown.

6. The system (10) according to claim 5, wherein the photosynthetic photon flux density at another tower peripheral portion (27) being illuminated at the given emulated time period is substantially equal.

7. The system (10) according to claim 5, wherein the predetermined number and sequence of light elements (33) are repeated along the height of the light post (6a-h) for all other segments (31).

8. The system (10) according to claim 1, further comprising a dosage pump (124) for delivering a predetermined amount of nutrients needed by the type of plant (19) being cultivated, and a controller (135) in data communication with said dosage pump (124) which is operable to regulate conductivity and pH of the irrigation solution deliverable to the irrigation means at predetermined times.

9. The system (10) according to claim 8, wherein the indoor facility is isolated from outdoor conditions present outwardly from the facility.

10. The system (10) according to claim 9, further comprising an artificial pollination system (170) for facilitating production of fruit and seed crops within the indoor facility.

11. The system (10) according to claim 10, wherein the artificial pollination system (170) Z comprises a plurality of vertically spaced air discharge nozzles (173) carried by each of the light posts, a pulsed supply of compressed air received in parallel by said nozzles (173) from an air receiver tank (177) serving to induce release of pollen from an anther of plants (19) being instantaneously positioned in proximity of said nozzles (173).

12. The system (10) according to claim 11, wherein the artificial pollination system (170) Z further comprises a conduit (172) external to the light post (176) which extends from the air tank (177) and is in fluid communication with each of the nozzles (173), and a control valve (179) operatively connected with said conduit (172) and in data communication with the controller (135) by which pressure of the compressed air is regulated.

13. The system (10) according to claim 12, wherein the artificial pollination system (170) further comprises a unit for controlling local air humidity in the vicinity of each tower (2a-d) of the module (5), the controller (135) being operable to:
a) command said unit to significantly reduce the local humidity to a level that ensures sufficient reduction in adhesiveness of the pollen for supporting pollen release;
b) receive information from a data source associated with the drive unit as to when a given plant (19) will be positioned in pollen releasable proximity to the nozzle (173);
c) command opening of the control valve (179) for a predetermined time so that the compressed air will be directed to said given plant;
d) command said unit to significantly increase the local humidity following the pollen release to ensure pollen viability; and
e) close the control valve (179) at the conclusion of a pollination cycle.

14. The system (10) according to claim 13, wherein the unit is an air conditioner and a dehumidifier (139).

15. The system (10) according to claim 1, further comprising a ceiling fan (162) centrally positioned within, and above, the darkened interior region (D) of the module (5), to cause, when operated, plant-released carbon dioxide to be transported to an outer region of the module (5) which is **characterized by** morning light conditions, noon light conditions, or afternoon light conditions, to induce an increased rate of plant growth as a result of absorbing a corresponding increased amount of carbon dioxide.

16. The system (10) according to claim 1, further comprising an open-loop recycling system (110) for reusing surplus irrigation fluid not consumed by plant roots.

17. The system (10) according to claim 1, wherein the light elements (33) mounted on each of the light posts (6a-h) are in continual operation and are cooled by mean of a cooling liquid introduced through a light post interior.

18. The system (10) according to claim 17, wherein the cooling liquid is the irrigation fluid.

19. The system (10) according to claim 1, wherein the light elements (33) mounted on each of the light posts (6a-h) are modulated to stimulate an improvement in plant metabolic processes.

20. The system (10) according to claim 1, further comprising, in the darkened interior region (D) a multidirectional pesticide spraying column (231) fed by a pesticide delivery conduit, conduit (237), said spraying column (231) having a plurality of nozzles (234) that protrude in different directions and being directed to one or more towers (2a-d) and being adapted to spray a predetermined amount of pesticides.

21. The system (10) according to claim 1, wherein, for one of the modules 5), an instantaneous illumination signature of first light posts simulates lighting conditions of morning at a first region, an instantaneous illumination signature of second light posts simulates lighting conditions of afternoon or evening at a second region, and an instantaneous illumination signature of third light posts simulates lighting conditions of noontime at a third region.

## Patentansprüche

1. Erdloses Indoor-Pflanzenanbausystem (10) zum Emulieren von Wachstumsbedingungen im Freien, umfassend:
a) eine Vielzahl von stationären Lichtsäulen (6a-h), die jeweils eingerichtet sind, einen vorbestimmten Sektor einer Indoor-Anlage gemäß einer vorbestimmten Beleuchtungssignatur zu beleuchten;
b) eine Vielzahl von Pflanzenwachstumstürmen (2a-c), welche dem Licht ausgesetzt werden können, das zu jedem Zeitpunkt von einer oder von mehreren der Lichtsäulen (6a-h) erzeugt wird, und welche angeordnet sind durch mindestens ein Modul (5), das einen vom Modul verdunkelten Innenbereich (D) definiert, innerhalb dessen momentan platzierte Pflanzen (19) eine Empfindung von Nachtzeit erhalten;
c) eine Vielzahl von Lichtelementen, die auf jeder der Lichtsäulen (6a-h) angebracht sind, wobei jedes der Lichtelemente mit einer direktionalen Linse (9) versehen ist, welche konfiguriert ist, einen Lichtabstrahlwinkel zu erzeugen, dessen Winkelbegrenzungen auf einen Turm- (2a-d) Umfang scheint, sodass als ein Ergebnis dessen Aufscheinens auf den Turm (2a-d) eine weitere Ausbreitung des abgestrahlten Lichts in den Innenbereich (2) des Moduls (5) zwischen zwei benachbarten Türmen (2a-d) blockiert wird, sodass dadurch sichergestellt wird, dass der Innenbereich (D) auf ein Strahlungsniveau abgedunkelt wird, das geringer ist als ein vorbestimmtes photosynthetisch aktives Strahlungsniveau für die angebaute Pflanze;
d) eine Antriebseinheit, welche konfiguriert ist, jeden der Türme (2a-d) zyklisch um eine im Wesentliche senkrechte Achse zu drehen um eine vollständige Turmdrehung einmal alle 24 Stunden, so dass dieser nacheinander den morgendlichen Lichtverhältnissen, den mittäglichen Lichtverhältnissen, den nachmittäglichen Lichtverhältnissen und den nächtlichen Verhältnissen ausgesetzt wird, in Übereinstimmung mit der Beleuchtungssignatur, die von den Lichtsäulen (6a-h) des mindestens einen Moduls (5) emittiert wird; und
e) Bewässerungsmittel zur Versorgung der Pflanzen (19), die in jedem der Türme (2a-d) angebaut werden, mit einer nährstoffreichen Lösung.

2. System (10) nach Anspruch 1, wobei jeder der Türme (2a-d) mit einer Vielzahl von Anbringungselementen konfiguriert ist, durch welche jeweils eine entsprechende Pflanze (19) an einem anderen Turmumfangsabschnitt (27) angebracht werden kann und dazu gedrängt wird, von dem Umfangsabschnitt nach außen hin zu wachsen, wobei Gruppen der Anbringungselemente an unterschiedlichen Höhenniveaus des Turms (2a-d) definiert sind.

3. System (10) nach Anspruch 2, wobei Blätter aller Pflanzen (19), die auf einem der Türme (2a-d) angebaut werden, einer im Wesentlichen gleichförmigen Verteilung von Licht, das von den Lichtelementen (33), die auf einer benachbarten der Lichtsäulen (6a-h) angebracht sind, abgestrahlt wird, ausgesetzt sind für eine gegebene emulierte Zeitdauer, ungeachtet eines Höhenunterschieds zwischen den Pflanzen (19).

4. System (10) nach Anspruch 3, wobei die Lichtelemente (33) ausreichend klein sind, so dass sie eine Dichte von nicht weniger als 40 Lichtelemente (33) in einer Lichtsäulenhöhe von 50 cm aufweisen und auf jeder der Lichtsäulen (6a-h) so angebracht sind, dass auf jeder gegebenen Höhe nur ein Lichtelement (33) angebracht ist.

5. System (10) nach Anspruch 4, wobei ein Segment (31) der Lichtelemente (33) eine vorbestimmte Anzahl und Reihenfolge von Lichtelementen (33) aufweist, die so angeordnet sind, dass konstituierende Strahlen, die von den Lichtelementen (33) des Segments (31) abgestrahlt werden, innerhalb eines konischen Verteilungswinkels gemischt werden, um eine photosynthetische Photonenflussdichte an dem Turmumfangsabschnitt (27), auf den der gemischte Strahl auftrifft, bereitzustellen, welche Photosynthese für eine gegebene Pflanze (19), die angebaut wird, zu stimulieren.

6. System (10) nach Anspruch 5, wobei die photosynthetische Photonenflussdichte an einem anderen Turmumfangsabschnitt (27), der bei der gegebenen emulierten Zeitperiode beleuchtet wird, im Wesentlichen gleich ist.

7. System (10) nach Anspruch 5, wobei die vorbestimmte Anzahl und Reihenfolge von Lichtelementen (33) sich wiederholt entlang der Höhe der Lichtsäule (6a-h) für alle anderen Segmente (31).

8. System (10) nach Anspruch 1, weiter umfassend eine Dosierpumpe (124) zum Abgeben einer vorbestimmten Menge von Nährstoffen, die von der Art von Pflanze (19), die angebaut wird, benötigt wird, und eine in Datenkommunikation mit der Dosierpumpe (124) stehende Steuer- und/oder Regeleinheit (135), die eingerichtet ist, eine Leitfähigkeit und einen pH der Bewässerungslösung, die an die Bewässerungsmittel abzugeben ist, zu vorgegebenen Zeiten zu regulieren.

9. System (10) nach Anspruch 8, wobei die Indoor-Anlage von Bedingungen im Freien isoliert ist, die außerhalb der Anlage herrschen.

10. System (10) nach Anspruch 9, weiter umfassend ein System (170) zur künstlichen Bestäubung zum Ermöglichen des Anbaus von Frucht- und Saatgutpflanzen in der Indoor-Anlage.

11. System (10) nach Anspruch 10, wobei das System (170) zur künstlichen Bestäubung eine Vielzahl von vertikal beabstandeten Luftauslassdüsen (173) aufweist, die von jeder der Lichtsäulen getragen werden, wobei die Düsen (173) parallel eine pulsierende Zufuhr von Druckluft von einem Luftempfangstank (177) erhalten, welche dazu dient, die Freisetzung von Pollen aus einem Staubbeutel von Pflanzen zu veranlassen, die momentan in der Nähe der Düsen (173) platziert sind.

12. System (10) nach Anspruch 11, wobei das System (170) zur künstlichen Bestäubung weiter eine Leitung (172) extern der Lichtsäule (176) aufweist, welche sich von dem Lufttank (177) erstreckt und in fluider Verbindung mit jeder der Düsen (173), steht, sowie ein Steuerventil (179), das operativ mit der Leitung (172) und in Datenkommunikation mit der Steuer- und/oder Regeleinheit (135) verbunden ist, durch welche der Druck der Druckluft reguliert wird.

13. System (10) nach Anspruch 12, wobei das System (170) zur künstlichen Bestäubung weiter eine Einheit zum Steuern und/oder Regeln von lokaler Luftfeuchte in der Nähe jedes Turms (2a-d) des Moduls (5) aufweist,
wobei die Steuer- und/oder Regeleinheit eingerichtet ist zum:
a) Ansteuern der Einheit, die lokale Feuchte signifikant zu reduzieren auf ein Niveau, welches eine ausreichende Verringerung in der Adhäsivität der Pollen sicherzustellen, um die Pollenfreisetzung zu unterstützen;
b) Empfangen von einer Datenquelle, die mit der Antriebseinheit assoziiert ist, von Information darüber, wenn eine gegebene Pflanze (19) in einer zur Pollenfreisetzung möglichen Nähe zu der Düse (173) positioniert sein wird;
c) Ansteuern des Öffnens des Steuerventils (179) für eine vorbestimmte Zeit, so dass die Druckluft auf die gegebene Pflanze (19) gerichtet wird;
d) Ansteuern der Einheit, die lokale Feuchte nach der Pollenfreisetzung signifikant zu erhöhen, um die Lebensfähigkeit der Pollen sicherzustellen; und
e) Schließen des Steuerventils (179) am Ende eines Bestäubungszyklus.

14. System nach Anspruch 13, wobei die Einheit eine Klimaanlage und Luftentfeuchter (139) ist.

15. System nach Anspruch 1, weiter umfassend einen Deckenlüfter (162), der mittig positioniert ist innerhalb und über dem abgedunkelten Innenbereich (D) des Moduls (5), um, wenn betrieben, dafür zu sorgen, dass von Pflanzen freigesetztes Kohlendioxid zu einem Außenbereich des Moduls (5) transportiert wird, der durch morgendliche Lichtverhältnisse, mittägliche Lichtverhältnisse oder nachmittägliche Lichtverhältnisse gekennzeichnet ist, um eine erhöhte Pflanzenwachstumsrate zu bewirken als ein Ergebnis dessen, eine entsprechend erhöhte Menge von Kohlendioxid aufzunehmen.

16. System (10) nach Anspruch 1, weiter umfassend ein Open-Loop Recyclingsystem (110) zum Wiederverwerten von überschüssiger Bewässerungsflüssigkeit, die nicht von Pflanzenwurzeln aufgenommen wird.

17. System (10) nach Anspruch 1, wobei die Lichtelemente (33), die auf jeder der Lichtsäulen (6a-h) angebracht sind, im Dauerbetrieb sind und mittels einer Kühlflüssigkeit gekühlt werden, welche durch ein Lichtsäuleninneres eingeführt wird.

18. System (10) nach Anspruch 17, wobei die Kühlflüssigkeit die Bewässerungsflüssigkeit ist.

19. System (10) nach Anspruch 1, wobei die Lichtelemente (33), die auf jeder der Lichtsäulen (6a-h) angebracht sind, moduliert werden, um eine Verbesserung in pflanzlichen Stoffwechselprozessen anzuregen.

20. System (10) nach Anspruch 1, außerdem umfassend, in dem abgedunkelten Innenbereich (D) eine multidirektionale Pestizidsprühsäule (231), welche von einer Pestizidzufuhrleitung, Leitung (237), gespeist wird, wobei die Sprühsäule (231) eine Vielzahl von Düsen (234) aufweist, die in unterschiedliche Richtungen vorstehen und zu einem oder mehreren Türmen (2a-d) gerichtet sind und ausgelegt sind, eine vorbestimmte Menge von Pestiziden zu sprühen.

21. System (10) nach Anspruch 1, wobei für eines der Module (5) eine Instantanbeleuchtungssignatur von ersten Lichtsäulen morgentliche Lichtbedingungen in einem ersten Bereich simuliert, eine Instantanbeleuchtungssignatur von zweiten Lichtsäulen nachmittägliche oder abendliche Lichtbedingungen in einem zweiten Bereich simuliert und eine Instantanbeleuchtungssignatur von dritten Lichtsäulen mittägliche Lichtbedingungen in einem dritten Bereich simuliert.

## Revendications

1. Système de culture de plantes hors-sol en intérieur (10) pour imiter les conditions de croissance extérieures, comprenant :
a) une pluralité de postes de lumière stationnaires (6a-h), dont chacun est adapté pour éclairer un secteur prédéterminé d'une installation en intérieur en fonction d'une signature d'éclairage prédéterminée ;
b) une pluralité de tours de croissance de plantes (2a-c) qui peuvent être exposées à la lumière générée à n'importe quel moment donné par un ou plusieurs des postes de lumière (6a-h) et qui sont agencées par au moins un module (5) définissant une région intérieure obscurcie de module (D) à l'intérieur de laquelle les plantes (19) positionnées instantanément reçoivent une sensation de nuit ;
c) une pluralité d'éléments de lumière montés sur chacun desdits postes de lumière (6a-h), dans lequel chacun desdits éléments de lumière est doté d'une lentille directionnelle (9) configurée pour produire un angle d'émission de lumière dont les limites angulaires sont incidentes sur la périphérie d'une tour (2a-d), provoquant le blocage de la propagation de la lumière émise correspondante jusqu'à ladite région intérieure (D) du module (5) entre deux tours (2a-d) adjacentes du fait de son incidence sur la périphérie de la tour (2a-d), pour garantir ainsi que ladite région intérieure (D) soit obscurcie à un niveau de rayonnement inférieur à un niveau de rayonnement photosynthétiquement actif prédéterminé pour la plante (19) étant cultivée ;
d) une unité d'entraînement configurée pour faire tourner cycliquement chacune desdites tours (2a-d) autour d'un axe sensiblement vertical d'une rotation de tour complète une fois toutes les 24 heures de sorte qu'elle soit successivement exposée à des conditions de lumière du matin, à des conditions de lumière du midi, à des conditions de lumière de l'après-midi et à des conditions de nuit en fonction de la signature d'éclairage émise par les postes de lumière (6a-h) de l'au moins un module (5) ; et
e) des moyens d'irrigation pour alimenter les plantes (19) étant cultivées dans chacune desdites tours (2a-d) en une solution riche en nutriments.

2. Système (10) selon la revendication 1, dans lequel chacune des tours (2a-d) est configurée avec une pluralité d'éléments de montage par chacun desquels une plante (19) correspondante est montable sur une portion périphérique de tour (27) différente et est amenée à pousser vers l'extérieur depuis ladite portion périphérique, des groupes desdits éléments de montage étant définis à différents niveaux de hauteur de la tour (2ad).

3. Système (10) selon la revendication 2, dans lequel les feuilles de toutes les plantes (19) étant cultivées sur une des tours (2a-d) sont exposées à une distribution sensiblement uniforme de la lumière émise par les éléments de lumière (33) montés sur un poste adjacent des postes de lumière (6a-h) pendant une durée imitée donnée malgré une différence de hauteur entre les plantes (19).

4. Système (10) selon la revendication 3, dans lequel les éléments de lumière (33) sont suffisamment petits pour qu'ils aient une densité de pas moins de 40 éléments de lumière (33) dans une hauteur de poste de lumière de 50 cm et soient montés sur chacun des postes de lumière (6a-h) de sorte que seul un élément de lumière (33) soit monté à une quelconque hauteur donnée.

5. Système (10) selon la revendication 4, dans lequel un segment (31) des éléments de lumière (33) présente un nombre et une séquence prédéterminés d'éléments de lumière (33) agencés de sorte que des faisceaux constitutifs émis par les éléments de lumière (33) dudit segment (31) soient mélangés dans un angle de distribution conique pour fournir une densité de flux de photons photosynthétiques à la portion périphérique de tour (27) frappée par le faisceau mélangé qui stimule la photosynthèse pour une plante (19) donnée étant cultivée.

6. Système (10) selon la revendication 5, dans lequel la densité de flux de photons photosynthétiques à une autre portion périphérique de tour (27) étant éclairée à la période imitée donnée est sensiblement égale.

7. Système (10) selon la revendication 5, dans lequel le nombre et la séquence prédéterminés d'éléments de lumière (33) sont répétés le long de la hauteur du poste de lumière (6a-h) pour tous les autres segments (31).

8. Système (10) selon la revendication 1, comprenant en outre une pompe de dosage (124) pour délivrer une quantité prédéterminée de nutriments nécessaires au type de plante (19) étant cultivé, et un dispositif de commande (135) en communication de données avec ladite pompe de dosage (124) qui est utilisable pour réguler la conductivité et le pH de la solution d'irrigation délivrable aux moyens d'irrigation à des moments prédéterminés.

9. Système (10) selon la revendication 8, dans lequel l'installation en intérieur est isolée des conditions extérieures présentes à l'extérieur de l'installation.

10. Système (10) selon la revendication 9, comprenant en outre un système de pollinisation artificiel (170) pour faciliter la production de récoltes de fruits et de graines à l'intérieur de l'installation en intérieur.

11. Système (10) selon la revendication 10, dans lequel le système de pollinisation artificiel (170) comprend une pluralité de buses de sortie d'air (173) espacées verticalement portées par chacun des postes de lumière, une alimentation pulsée en air comprimé reçue en parallèle par lesdites buses (173) à partir d'un réservoir de réception d'air (177) servant à induire la libération de pollen d'une anthère de plantes (19) étant positionnées instantanément à proximité desdites buses (173).

12. Système (10) selon la revendication 11, dans lequel le système de pollinisation artificiel (170) comprend en outre un conduit (172) externe au poste de lumière (176) qui s'étend depuis le réservoir d'air (177) et est en communication fluidique avec chacune des buses (173), et une vanne de commande (179) connectée de manière utilisable audit conduit (172) et en communication de données avec le dispositif de commande (135) par lequel la pression de l'air comprimé est régulée.

13. Système (10) selon la revendication 12, dans lequel le système de pollinisation artificiel (170) comprend en outre une unité pour commander l'humidité de l'air locale à proximité de chaque tour (2a-d) du module (5), le dispositif de commande (135) étant utilisable pour :
a) ordonner à ladite unité de réduire sensiblement l'humidité locale à un niveau qui garantisse une réduction suffisante de l'adhérence du pollen pour soutenir la libération de pollen ;
b) recevoir des informations d'une source de données associée à l'unité d'entraînement quant au moment où une plante (19) donnée sera positionnée à une proximité de libération de pollen de la buse (173) ;
c) ordonner l'ouverture de la vanne de commande (179) pendant une durée prédéterminée de sorte que l'air comprimé soit envoyé à ladite plante donnée ;
d) ordonner à ladite unité d'augmenter sensiblement l'humidité locale après la libération de pollen pour garantir la viabilité du pollen ; et
e) fermer la vanne de commande (179) à la conclusion d'un cycle de pollinisation.

14. Système (10) selon la revendication 13, dans lequel l'unité est un climatiseur et un déshumidificateur (139).

15. Système (10) selon la revendication 1, comprenant en outre un ventilateur de plafond (162) positionné centralement dans, et au-dessus de la région intérieure obscurcie (D) du module (5), pour provoquer, lorsqu'il est utilisé, le transport du dioxyde de carbone libéré par les plantes jusqu'à une région extérieure du module (5) qui est **caractérisée par** des conditions de lumière du matin, des conditions de lumière du midi, ou des conditions de lumière de l'après-midi pour induire un taux accru de croissance des plantes du fait de l'absorption d'une quantité accrue correspondante de dioxyde de carbone.

16. Système (10) selon la revendication 1, comprenant en outre un système de recyclage à boucle ouverte (110) pour réutiliser le surplus de fluide d'irrigation non consommé par les racines des plantes.

17. Système (10) selon la revendication 1, dans lequel les éléments de lumière (33) montés sur chacun des postes de lumière (6a-h) sont en utilisation continue et sont refroidis au moyen d'un liquide de refroidissement introduit à travers un intérieur de poste de lumière.

18. Système (10) selon la revendication 17, dans lequel le liquide de refroidissement est le fluide d'irrigation.

19. Système (10) selon la revendication 1, dans lequel les éléments de lumière (33) montés sur chacun des postes de lumière (6a-h) sont modulés pour stimuler une amélioration des processus métaboliques des plantes.

20. Système (10) selon la revendication 1, comprenant en outre, dans la région intérieure obscurcie (D), une colonne de pulvérisation de pesticide multidirectionnelle (231) alimentée par un conduit de délivrance de pesticide (237), ladite colonne de pulvérisation (231) ayant une pluralité de buses (234) qui font saillie dans différentes directions et étant dirigées vers une ou plusieurs tours (2a-d) et étant adaptées pour pulvériser une quantité prédéterminée de pesticides.

21. Système (10) selon la revendication 1, dans lequel, pour un des modules (5), une signature d'éclairage instantanée de premiers postes de lumière simule des conditions d'éclairage du matin au niveau d'une première région, une signature d'éclairage instantanée de deuxièmes postes de lumière simule des conditions d'éclairage de l'après-midi ou du soir au niveau d'une deuxième région, et une signature d'éclairage instantanée de troisièmes postes de lumière simule des conditions d'éclairage du midi au niveau d'une troisième région.
